# EUROPÄISCHE PATENTSCHRIFT

(11) **EP 3 600 480 B1**
(45) Veröffentlichungstag und Bekanntmachung des Hinweises auf die Patenterteilung: **19.05.2021**
(21) Anmeldenummer: 18713837.5
(22) Anmeldetag: 20.03.2018
(51) Int. Cl.: A61M 1/14, A61M 1/36, H01L 41/113

(54) **MEDIZINISCHE EINRICHTUNG MIT ADDITIV AUFGEBRACHTEM WANDLER SAMT LEITERBAHN**
MEDICAL DEVICE WITH ADDITIVELY APPLIED CONVERTER TOGETHER WITH CONDUCTOR TRACK
ÉQUIPEMENT MÉDICAL DOTÉ D'UN TRANSDUCTEUR APPLIQUÉ PAR FABRICATION ADDITIVE ET D'UN TRACÉ CONDUCTEUR

(30) Priorität: 24.03.2017 DE 102017106403
(43) Veröffentlichungstag der Anmeldung: 05.02.2020
(73) Patentinhaber: Fresenius Medical Care Deutschland GmbH, 61352 Bad Homburg (DE)
(72) Erfinder: HEIDE, Alexander, 65817 Eppstein (DE); NIKOLIC, Dejan, 65812 Bad Soden (DE)
(74) Vertreter: Bobbert & Partner Patentanwälte PartmbB
(86) Internationale Anmeldenummer: PCT/EP2018/057011
(87) Internationale Veröffentlichungsnummer: WO 2018/172351

(56) Entgegenhaltungen:
- US-A1- 2009 012 452
- US-A1- 2011 214 504

## Beschreibung

Die vorliegende Erfindung betrifft eine medizinische Einrichtung gemäß dem Oberbegriff des Anspruchs 1. Sie betrifft ferner ein Herstellverfahren für eine medizinische Einrichtung gemäß dem Oberbegriff des Anspruchs 8.

Bekannte Blutbehandlungsvorrichtungen werden zur Blutbehandlung mit wenigstens einer medizinischen Einrichtung verbunden, beispielsweise in Gestalt einer Blutkassette, in welcher Blut behandelt oder vorübergehend gespeichert wird. Als Blutkassetten ausgestaltete medizinische Einrichtungen sind aus der DE 10 2009 018 664 A1 bekannt.

In der US 2011/0214504 A1 sind Einwegdrucksensorsysteme und Packarten hierzu offenbart.

Aus der US 2009/012452 A1 sind Dialysierflüssigkeitsmesssysteme mit leitenden Sensoren bekannt.

Aufgabe der vorliegenden Erfindung ist, eine weitere medizinische Einrichtung anzugeben. Ferner soll ein Herstellverfahren für eine solche Einrichtung vorgeschlagen werden.

Diese Aufgabe kann durch eine medizinische Einrichtung mit den Merkmalen des Anspruchs 1 und durch ein Verfahren mit den Merkmalen des Anspruchs 8 gelöst werden.

Erfindungsgemäß wird somit eine medizinische Einrichtung mit wenigstens einem Hartteil mit vollständigen oder unvollständigen Fluidbahnen zum Führen eines medizinischen Fluids, insbesondere Blut, im Hartteil oder durch das Hartteil hindurch, angegeben.

Die medizinische Einrichtung weist ferner wenigstens einen Wandler auf. Der Wandler ist angeordnet zum Messen einer Eigenschaft des medizinischen Fluids oder eines anderen Fluids, während dieses in einer der Fluidbahnen vorliegt. Alternativ oder ergänzend ist der Wandler angeordnet zum Messen einer Eigenschaft der medizinischen Einrichtung oder einer Einwirkung wie einen von außen oder innen aufgebrachten Druck auf letztere.

Der Wandler ist zumindest in einem ersten Abschnitt hiervon mittels eines additiven Aufbringungsverfahrens, vorzugsweise eines Druckverfahrens - direkt oder indirekt - auf das Hartteil aufgebracht.

Die medizinische Einrichtung weist ferner wenigstens eine Leiterbahn auf.

Wenigstens ein zweiter Abschnitt des Wandlers oder der Leiterbahn ist mittels eines zweiten additiven Aufbringungsverfahrens auf das Hartteil aufgebracht.

Das erste additive und das zweite additive Aufbringungsverfahren unterscheiden sich voneinander.

Das erfindungsgemäße Verfahren dient insbesondere dem Herstellen einer erfindungsgemäßen medizinischen Einrichtung. Es umfasst das Herstellen oder Bereitstellen eines Hartteils der medizinischen Einrichtung, welches Fluidbahnen, ein Fluidsystem, oder Abschnitte hierfür, für ein medizinisches Fluid aufweist.

Das Verfahren umfasst ferner das Aufbringen wenigstens eines ersten Abschnitts eines Wandlers oder einer Leiterbahn mittels eines ersten additiven Aufbringungsverfahrens, vorzugsweise eines Druckverfahrens, auf das Hartteil.

Das Verfahren umfasst ferner das Aufbringen wenigstens eines zweiten Abschnitts des Wandlers oder der Leiterbahn mittels eines zweiten additiven Aufbringungsverfahrens auf das Hartteil.

Die erfindungsgemäße Behandlungsvorrichtung weist wenigstens einen Multipolverbinder zum Verbinden mit dem Multipolstecker (oder "Multistecker" als

Austauschbegriff für "Multipolstecker" im Rahmen der vorliegenden Erfindung) einer medizinischen Einrichtung auf.

Die erfindungsgemäße Behandlungsvorrichtung kann konfiguriert sein zum Empfangen und Verarbeiten der mittels des Multipolverbinders empfangenen Signale.

Die erfindungsgemäße Behandlungsvorrichtung kann mit einer erfindungsgemäßen medizinischen Einrichtung verbunden sein.

Bei allen Ausführungen hierin ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Erfindungsgemäße Ausführungsformen können eines oder mehrere der vorstehend oder im Folgenden genannten Merkmale aufweisen. Dabei können die hierin genannten Merkmale in beliebiger Kombinationen Gegenstand von erfindungsgemäßen Ausführungsformen sein, sofern der Fachmann eine konkrete Kombination nicht als technisch unmöglich erkennt. Erfindungsgemäße Ausführungsformen sind ferner Gegenstand der Unteransprüche.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

In einigen, beispielhaften erfindungsgemäßen Ausführungsformen sind unter dem Hartteil (hierin auch als Hartkörper oder als Substrat bezeichnet) der in der Regel in einem Spritzgussverfahren hergestellte und daher hierin als "hart" bezeichnete Korpus der medizinischen Einrichtung, welcher mit einer vergleichsweise "weichen" Folie bedeckt sein kann, und/oder seine Anhänge wie Schläuche usw., zu verstehen. Das Hartteil kann aus PP (Polypropylen), PE (Polyethylen), PA, ABS, PMMA, PC, PVC oder anderen dem Fachmann hinlänglich bekannten Polymeren oder anderen Materialien gefertigt sein. Es kann aus Isolatormaterialien, wie insbesondere z. B. Keramik, gefertigt sein.

Im Rahmen der vorliegenden Beschreibung werden die Begriffe "Sensor" und "Sensoranordnung" gleichbedeutend für ein System mit oder aus einem Wandler, Signalübertragungskomponenten und Auswerteeinheit verwendet. Ein Wandler ist dabei vorzugsweise als ein einrichtungsseitig vorgesehener Abschnitt des Sensors zu verstehen, der mit dem Messmedium in Kontakt steht. Er wandelt das Ergebnis seiner Messung in geeignete Signale, z. B. Strom- oder Spannungssignale um. Die ebenfalls zur Sensoranordnung zählenden Signalübertragungskomponenten leiten das Ergebnis seiner Messung an die Auswerteeinheit weiter, die im Stand der Technik üblicherweise maschinenseitig vorgesehen ist.

Unter einem "Leitungssegment" im Sinne der vorliegenden Beschreibung ist in einigen erfindungsgemäßen Ausführungsformen eine lokale Umgebung mit jeweils spezifischen Bedingungen bzgl. des Materials, der Topologie, der Funktion in der medizinischen Vorrichtung sowie der räumlichen Ausdehnung zu verstehen.

Der Begriff "Leitungssegment" kann eine Abfolge oder ein Aufeinanderfolgen unterschiedlicher Strukturen von zur Leitung verwendeter Elemente darstellen. Ein Leitungssegment kann ein Abschnitt einer Gesamtleitung sein, eine Leiterbahn, ein Leitungsabschnitt oder dergleichen.

So können z. B. an unterschiedlichen Stellen eines Disposables unterschiedliche Voraussetzungen an die Leitungsführung oder die Strukturen zum Erzeugen und/oder Leiten eines Signals erfüllt sein müssen. Daher kann es von Vorteil sein, unterschiedliche technische Lösungen für den jeweiligen Leitungsabschnitt auszuwählen. Diese technischen Lösungen können im Einzelnen möglicherweise vorbekannt sein. Erfindungsgemäß werden sie auf günstige Weise kombiniert. So können innerhalb einer Leitungsstrecke verschiedene Anforderungsbereiche ("Leitungssegmente") vorliegen, für welche jeweils adäquate technische Lösungen (z. B. Durchkontaktierung zum Sensor, Siebdruck für die flächige Verbindung, 3D und maskierungsfrei am Multipolstecker) ausgewählt sind.

Die aufgebrachten Bahnen/Strukturen haben z. T. unterschiedliche Aufgaben, obwohl sie alle im weitesten Sinne an sich nur "Bahnen" sind. Die jeweiligen Abschnitte sind hierin jeweils als Leitungssegment bezeichnet. So kann z. B. das erste Segment (das an sich wiederum nur eine "Bahn" sein kann) Dehnung in eine Widerstandsänderung wandeln, was sich in einem Signal niederschlagen kann. Diese Bahn wird eher sehr dünn, mäanderförmig flexibel und optional auf einer räumlich gekrümmten Oberfläche vorgesehen sein. Bestimmte Prozessparameter können hierfür grundlegend vorgesehen sein, z. T. elementar sein (etwa die Verwendung von Tinte, die Dimension, das Dehnungsverhalten, die elektrische Isolation, die Abschirmung (EMV) etc.).

In einem zweiten Leitungssegment geht es optional nur noch um den "Transport" oder die Leitung des Signals zu einem "Sammelpunkt" (welcher wiederum als drittes Leitungssegment verstanden werden kann). Die jeweiligen Eigenschaften dieses zweiten bzw. dieses dritten Leitungssegments können sich grundlegend voneinander unterscheiden. Sie müssen z. B. nicht mehr 3-dimensional gekrümmt sein und könnten daher auch mittels Siebdruck o. ä. aufgebracht/angebunden werden. Gleiches gilt für den "Sammelpunkt". Diese Eigenschaften werden durch eine geänderte Robustheit gegen mechanische Kontaktierung bestimmt (z. B. dicker, fester...).

Ein erstes Leitungssegment stellt beispielsweise zunächst der Wandler selbst dar. Hier wird an einem bestimmten Punkt, oder in der lokalen Umgebung eines bestimmten Punktes, die zu messende Größe aufgenommen und in eine elektro-magnetisch übertragbare Größe gewandelt. Diese Größe wird zunächst aus der lokalen Umgebung des Messpunktes herausgeleitet.

Ein zweites Leitungssegment stellt beispielsweise die zumeist flächige Leitung über die medizinische Einrichtung hinweg, z. B. zum Multipolstecker hin, dar.

Ein drittes Leitungssegment stellt exemplarisch der Multipolstecker selbst und seine Verbindung zur Maschinenseite dar. Eine erste Konnektierung kann also beispielsweise zwischen dem ersten Leitungssegment (Sensor/Wandler) und dem zweiten Leitungssegment (flächige Signalleitung) erfolgen. Eine zweite Konnektierung kann also beispielsweise zwischen dem zweiten Leitungssegment (flächige Signalleitung) und dem dritten Leitungssegment (Multipolstecker) erfolgen.

In einigen beispielhaften, erfindungsgemäßen Ausführungsformen umfasst das Aufbringungsverfahren das Aufbringen von leitfähiger Tinte oder besteht hieraus.

Unter leitfähiger Tinte sind hier Fluide mit beispielsweise Nano- oder Mikropartikeln zu verstehen, die, wenn sie geeignet dicht zusammen aufgebracht werden, eine funktionale Bahn ergeben, wie z. B. eine Leiterbahn (hierin auch als Elektrode bezeichnet), über die unter anderem Signale übertragen werden können. Im Sinne der Erfindung kommen aber auch andere Materialien und Aggregatszustände als leitfähige Tinte in Frage oder zählen hierzu. Leitfähige Tinte kann idealerweise auch biokompatibel sein, muss aber nicht. Es können ergänzend oder alternativ lebende Zellen, Proteine etc. Bestandteil der durch sie leitfähig gemachten Tinte sein.

Die leitfähige Tinte oder das alternative Material kann in beliebigen Aggregatszuständen verwendet werden. Unter einem Aggregatszustand im Sinne der vorliegenden Erfindung kann in einigen Ausführungsformen ein festes oder flüssiges Hydrogel verstanden werden, in welches ein Biomarker eingelagert ist. Es können auch gefrorene (also feste) Substanzen oder mikroinkapsulierte Wirkstoffe/Reagenzien verarbeitet werden. Auch können Bahnen per Sublimation/Kondensation aus der Gasphase abgeschieden werden.

Unter leitfähiger Tinte sind hier beispielsweise auch Liquide zu verstehen, die Karbon, leitende Polymere, Metallpartikel und/oder Kombinationen hiervon aufweisen, ferner metallisierte Tinte.

Die aus dem Stand der Technik bekannte Technik des Aerosoljet-Druckens (siehe hierzu die EP 2 559 656 A1) kommt hier als Beispiel für ein additives Aufbringen und insbesondere zum Bedrucken in Betracht. Die dort offenbarte Technik und die darin beschriebenen Vorrichtungen zum Ausführen dieser Technik eignen sich hierfür, insbesondere auch aufgrund der geometrischen Ausführung der Druckdüse (Nozzle tip), welche die notwendige Anzahl an Freiheitsgraden in der Bewegung beim Druckvorgang erlaubt.

Die vorliegende Erfindung ist natürlich nicht auf die Anwendung des Aerosoljets begrenzt. Der Fachmann erkennt, dass alle additiven und/oder maskierungsfreien Druckverfahren, insbesondere mittels welcher leitfähige Tinte im Sinne dieser Beschreibung appliziert werden kann, von der vorliegenden Erfindung umfasst sind.

In einigen beispielhaften, erfindungsgemäßen Ausführungsformen umfasst ein Aufbringungsverfahren ein Aufbringen in mehreren Schichten.

In einigen beispielhaften, erfindungsgemäßen Ausführungsformen umfasst ein Aufbringungsverfahren eine Sequenz oder eine Aufeinanderfolge von eigenständigen Aufbringungsverfahren oder von Schritten, die zu ein und demselben Aufbringungsverfahren zählen.

In einigen beispielhaften, erfindungsgemäßen Ausführungsformen umfasst das additive Aufbringen sog. "additive manufacturing" bzw. generative Fertigungsverfahren.

Insbesondere können hierunter die folgenden Verfahren verstanden werden:
- selektives Laserschmelzen (SLM),
- selektives Lasersintern (SLS),
- Selective Heat Sintering (SHS),
- Binder Jetting (Verfestigen von Pulvermaterial mittels Binder),
- Elektronenstrahlschmelzen (Electron Beam Melting = EBM),
- Fused Deposition Modelling (FDM oder auch Fused Filament Fabrication (FFF)),
- Auftragschweißen bzw. Cladding,
- Wax Deposition Modelling (WDM),
- Contour Crafting,
- Metall-Pulver-Auftragsverfahren (MPA),
- Kaltgasspritzen
- Stereolithografie (SLA) + Mikro-SLA,
- Verfahren, welche Digital Light Processing (DLP) zur Belichtung nutzen und
- Liquid Composite Moulding (LCM),
- Laminated Object Modelling (LOM),
- 3D-Siebdruck von Metallen und
- Lichtgesteuerte Elektrophoretische Abscheidung.

In einigen beispielhaften, erfindungsgemäßen Ausführungsformen umfasst das Aufbringungsverfahren ein maskierungsfreies Aufbringen oder besteht hieraus. Dies kann auf das erste, das zweite und/oder weitere Aufbringungsverfahren zutreffen, sowie auf Kombinationen hiervon.

In einigen beispielhaften, erfindungsgemäßen Ausführungsformen ist unter einem maskierungsfreien Aufbringen ein Aufbringen ohne Maske oder Schablone zu verstehen.

In einigen beispielhaften, erfindungsgemäßen Ausführungsformen ist das Aufbringungsverfahren kein Maskentintendruck, kein *"ink stencil printing",* kein *"screen printing",* kein Fotolithographie-Verfahren, insbesondere jeweils nicht in einem kontinuierlichen Verfahren.

In einigen beispielhaften, erfindungsgemäßen Ausführungsformen ist unter einem maskierungsfreien Aufbringen ein Verzicht auf jede - anschließend zu entfernende - Hilfsschicht oder Blende zu verstehen.

Unter "Maskierung" kann hierin ein Abschatten oder Schützen in geeigneter Form von Bereichen, welche nicht beschichtet werden dürfen, verstanden werden. Diese Maskierung geschieht z. B. durch Blenden (z. B. Siebdruck, Sprühlackierung, etc.) oder durch "Lack"-Masken (z. B. Wafer, Platinen-Fertigung, etc.).

In einigen beispielhaften, erfindungsgemäßen Ausführungsformen weist die medizinische Einrichtung eine Vielzahl von Wandlern, d. h. zwei oder mehr, auf, welche, jeweils zumindest in einem Abschnitt hiervon, mittels eines additiven Aufbringungsverfahrens (oder mehrerer additiver Aufbringungsverfahren), vorzugsweise Druckverfahren(s), - direkt oder indirekt - auf das Hartteil aufgebracht sind.

In einigen beispielhaften, erfindungsgemäßen Ausführungsformen wurden additiv weitere Abschnitte einer Sensoranordnung oder einer Vielzahl von Sensoranordnungen aufgebracht, vorzugsweise unter Einsatz desselben Aufbringungsverfahrens.

In einigen beispielhaften, erfindungsgemäßen Ausführungsformen wurden die mittels eines additiven Aufbringungsverfahrens aufgebrachten Abschnitte der einen Sensoranordnung oder der Vielzahl der Sensoranordnungen im selben Produktionsschritt aufgebracht.

In einigen beispielhaften, erfindungsgemäßen Ausführungsformen umfassen die mittels eines additiven Aufbringungsverfahrens aufgebrachten Abschnitte zusätzlich zum wenigstens einen Wandler wenigstens Leiterbahnen, Elektroden, einen Multipolstecker oder jeweils eine Vielzahl hiervon.

In einigen beispielhaften, erfindungsgemäßen Ausführungsformen sind der oder die Wandler zum Messen oder Bestimmen von Leitfähigkeit, Konzentration, Druck, Spannung oder Strom ausgestaltet oder konfiguriert.

In einigen beispielhaften, erfindungsgemäßen Ausführungsformen bewirkt das Aufbringungsverfahren eine 2-dimensionale oder eine 3-dimensionale Aufbringung, und/oder die mittels eines additiven Aufbringungsverfahrens aufgebrachten Wandler und/oder weiteren Abschnitte wurden mittels des Aufbringungsverfahrens 2-dimensional oder 3-dimensional aufgebracht.

In einigen beispielhaften, erfindungsgemäßen Ausführungsformen wurde wenigstens ein Multipolstecker ebenfalls mit dem erstem Aufbringungsverfahren oder zeitgleich zu diesem aufgebracht.

In einigen beispielhaften, erfindungsgemäßen Ausführungsformen ist zum Aufbringen des mittels additiver Aufbringungsverfahren aufgebrachten ersten Abschnitts neben dem ersten Aufbringungsverfahren ein weiteres Aufbringungsverfahren, bei dem es sich nicht um das zweite Aufbringungsverfahren handelt, zum Einsatz gekommen; ein solcher Fertigungsschritt kann vom erfindungsgemäßen Verfahren umfasst sein.

In einigen beispielhaften, erfindungsgemäßen Ausführungsformen ist zum Aufbringen des mittels additiver Aufbringungsverfahren aufgebrachten zweiten Abschnitts neben dem zweiten Aufbringungsverfahren ein weiteres Aufbringungsverfahren, bei dem es sich nicht um das erste Aufbringungsverfahren handelt, zum Einsatz gekommen; ein solcher Fertigungsschritt kann vom erfindungsgemäßen Verfahren umfasst sein.

In einigen beispielhaften, erfindungsgemäßen Ausführungsformen wurden der erste, der zweite und/oder weitere Abschnitte mittels Spritzverfahrens aufgebracht, z. B. als Zwei-Komponentendruck, z. B. mittels leitfähiger Polymere.

In einigen beispielhaften, erfindungsgemäßen Ausführungsformen ist oder umfasst das additive Aufbringungsverfahren kein Kleben, Schrauben, Stecken, kein *screen-printing,* kein *tampon printing* und/oder verwendet keine Silberpaste, keine Aluminiumpaste, keine *"inkjet-produced patterns of solid copper".*

In einigen beispielhaften, erfindungsgemäßen Ausführungsformen hat oder ist der Sensor, oder der Wandler, keine Folie.

In einigen beispielhaften, erfindungsgemäßen Ausführungsformen weist die medizinische Einrichtung wenigstens einen Kontaktstift auf. Dieser liegt zumindest abschnittsweise im Hartteil vor und steht in elektrischer Leitverbindung mit dem Wandler oder einer mit dem Wandler verbundenen Leiterbahn.

Der Kontaktstift kann ein Metallstift oder Metallabschnitt sein oder jeweils einen solchen aufweisen.

Der Kontaktstift kann ein Spritzgussprodukt sein. Er kann z. B. mittels eines Zweikomponentenspritzgusses gefertigt worden sein. Er kann z. B. leitfähige Polymere, metallische Partikel oder andere elektrisch leitende Stoffe aufweisen.

Der Kontaktstift kann während der Fertigung des Hartteils oder im Anschluss daran in das Hartteil eingebracht werden oder worden sein.

In einigen beispielhaften, erfindungsgemäßen Ausführungsformen umfasst das Verfahren weiter wenigstens einen der folgenden Schritte: Nachbearbeitungsschritte, insbesondere an den Leiterbahnen oder Elektroden, wie z. B. Schleifen, Polieren, Isolieren, Aufbringen weiterer funktionaler Schichten aus anderem Material; additives Aufbringen, z. B. durch Drucken oder Bedrucken der Signalanbindung an die Maschinenschnittstelle, additives Aufbringen, z. B. durch Drucken oder Bedrucken des Multipolsteckers und/oder Zusammenfügen von zwei oder mehr mittels additiven Aufbringens bearbeiteter Abschnitte der medizinischen Einrichtung.

Der Kontaktstift kann während des Spritzgussverfahrens, mit welchem das Hartteil gefertigt wurde, in das Hartteil eingebunden werden oder worden sein.

In einigen beispielhaften, erfindungsgemäßen Ausführungsformen ist die medizinische Einrichtung eine Blutkassette. Sie kann zum einmaligen Einsatz bei der Blutbehandlung vorgesehen sein.

In einigen beispielhaften, erfindungsgemäßen Ausführungsformen umfasst das Verfahren das Aufbringen von wenigstens zwei Leiterbahnen, die sich in jeweils wenigstens einem Abschnitt kreuzen. Es umfasst ferner das Aufbringen einer Isolierschicht zwischen den beiden Leiterbahnen.

In einigen beispielhaften, erfindungsgemäßen Ausführungsformen umfasst das Verfahren das Aufbringen einer Abschirmschicht auf wenigstens einer der Leiterbahnen.

Sowohl die Isolierschicht als auch die Abschirmschicht können wie Leiterbahnen aufgebracht sein.

Sowohl die Leiterbahn (hierin auch als Signalleiter bezeichnet) als auch der Multipolstecker können planar (also 2-dimensional) oder 3-dimensional mit einer oder mehreren additiven und maskierungsfreien Drucktechniken aufgebracht werden. Auch hier sind ein oder mehrere Nachbearbeitungsschritte wie vorstehend bei den Leiterbahnen beschrieben, optional umfasst.

In einigen beispielhaften, erfindungsgemäßen Ausführungsformen umfasst das Verfahren weiter das Zusammenfügen von zwei oder mehr, wie hierin beschrieben, mittels additiven Aufbringens bearbeiteten Abschnitten oder Komponenten der medizinischen Einrichtung.

In einigen erfindungsgemäßen, beispielhaften Ausführungsformen ist die erfindungsgemäße medizinische Einrichtung ein Schlauch, ein Schlauchsystem, ein Schlauchset, eine Blutkassette oder jeweils ein Teil hiervon.

Ein "medizinisches Fluid" im Sinne der vorliegenden Erfindung schließt jede medizinische Flüssigkeit und/oder jedes medizinische Gas sowie beliebige Kombinationen ein. Vorzugsweise handelt es sich bei dem Fluid um Blut.

Eine erfindungsgemäße medizinische Einrichtung kann eine Einmalkomponente oder ein Einmalartikel ("Disposable") sein, welcher beispielsweise aus einem Kunststoffmaterial gefertigt ist.

Die erfindungsgemäße medizinische Einrichtung kann mittels eines Spritzgussverfahrens hergestellt sein.

Die erfindungsgemäße medizinische Einrichtung kann über Flüssigkeits- und/oder Gasanschlüsse, über halboffene Kanäle und/oder Kammern verfügen. Ein oder mehrere Abdeckungselemente, wie beispielsweise Membranen oder Folien, können für den Abschluss und/oder die Abdichtung der Kanäle und Kammern sorgen.

Die Blutbehandlung, für welche die medizinische Einrichtung verwendet wird, kann beispielsweise ein Dialysierverfahren, eine Hämodialyse, Hämofiltration, Hämodiafiltration und/oder dergleichen sein.

In bestimmten beispielhaften, erfindungsgemäßen Ausführungsformen ist die medizinische Einrichtung eine Blutkassette. Das Hartteil ist in diesen Fällen ein Kassettenkörper oder Kassettengrundkörper oder ein Schlauchabschnitt.

In bestimmten beispielhaften, erfindungsgemäßen Ausführungsformen weist die medizinische Einrichtung ferner wenigstens zwei Konnektoren für Pumpschlauchsegmente für peristaltische Pumpen, mit oder ohne den Pumpschlauchsegmenten, auf.

In bestimmten beispielhaften, erfindungsgemäßen Ausführungsformen weist die medizinische Einrichtung eine Single-Needle-Sterilmembran auf.

In bestimmten beispielhaften, erfindungsgemäßen Ausführungsformen weist die Vorrichtung Dehnungsmessstreifen (DMS Sensoren) auf. Die Verformung des Untergrundes führt zur Längenänderung der Messbahn oder des Leitungssegments und ändert deren Widerstand. Alternativ könnte auch ein Material mit piezoelektrischen Eigenschaften aufgedruckt werden z. B. PVDF (Polymer) oder Piezokeramik. Mit Hilfe des Piezoeffekts würden bevorzugt (schnelle) Druckänderungen oder Schwingungen gemessen.

In bestimmten beispielhaften, erfindungsgemäßen Ausführungsformen ist die Behandlungsvorrichtung ausgestaltet als Blutbehandlungsvorrichtung, insbesondere als Apheresevorrichtung oder Dialysevorrichtung, insbesondere als Hämofiltrationsvorrichtung, als Hämodiafiltrationsvorrichtung, als Filtrationsvorrichtung oder als Vorrichtung zum extrakorporalen Gasaustausch.

In bestimmten beispielhaften, erfindungsgemäßen Ausführungsformen weist die Behandlungsvorrichtung Aktuatoren wie z. B. Pumpen oder Ventile und/oder mechanische oder nicht-mechanische Schnittstellen zum Einwirken mittels dieser Aktuatoren auf die medizinische Einrichtung auf.

In bestimmten beispielhaften, erfindungsgemäßen Ausführungsformen umfasst der Multipolverbinder der Behandlungsvorrichtung eine Spannungsleitung, mittels welcher die medizinische Einrichtung mit elektrischer Spannung versorgt wird. Die elektrische Versorgung kann der Datenauslese oder dem Auslesen von Daten ebenso wie dem Betrieb der Sensoren der medizinischen Einrichtung dienen.

Beispiele für Wandler im Sinne der vorliegenden Erfindung umfassen insbesondere:
1) Wandler für kapazitive Messungen: Zwischen zwei leitenden Oberflächen wird ein Dielektrikum angebracht. Die zu messende Eigenschaft hat eine Wechselwirkung mit der Dielektrizitätskonstante des Dielektrikums und kann über die Kondensatoreigenschaften bestimmt werden. Mit dieser Art des Wandlers können u. a. bestimmt werden: Leitfähigkeit, Level (Füllstand bzw. Vorhandensein einer Flüssigkeit), Druck, Abstand (Näherungssensor).
2) Wandler für Widerstandsmessung: Äußere Wirkung auf einen Draht beeinflusst dessen Widerstand. Mit dieser Art des Wandlers können u.a. bestimmt werden:
   Temperatur, Druck (Dehnungsmessstreifen DMS), Gewicht, Kraft, Weg, Kontakt (ja, nein).
3) Wandler mittels Piezoeffekt: Ein Piezodot (z. B. PVDF) wird auf die Oberfläche einer Messzelle gedruckt. Messprinzip mittels Druckaufnahme, d. h. direktes Wandeln von äußerem Druck in Spannung, oder mittels Ultraschallerzeugung (räumliche Vermessung (Laufzeit) bzw. Dichtemessung (Amplitude) in Transmission mit einem zweiten Piezodot als Empfänger oder in Reflexion mit nur einem Piezodot). Mit dieser Art des Wandlers können u.a. bestimmt werden:
   Lufterkennung, Flussmessung, Bluterkennung, räumliche Vermessung (vgl. Unterwassermikrophone, Schrauben mit aufgebrachter Piezo-Drucksensorik zur Spannkraftüberwachung).
4) Magneto-induktive Wandler: Bekannter Disposable MID-Sensor zur Messung von Fluss oder Leitfähigkeit - in dieser Variante sind die Elektroden im Disposable nicht eingelegt und umspritzt, sondern aufgedruckt. Das Prinzip des Sensors ist z. B. in der WO2011/113838 beschrieben.
5) Optische Wandler: Ein Reagenz wird auf die Messoberfläche aufgedruckt. Sie wird entweder von außen oder von einem ebenfalls aufgedruckten optischen Emitter (Diode) bestrahlt. Die Eigenschaften des Reagenzes bezüglich der Reflexion, Absorption, Lumineszenz, Fluoreszenz sind von der zu vermessenden Größe abhängig. Über einen Fotodetektor, welcher die entsprechende Intensität aufnimmt, kann die Information gewonnen werden. Dabei kann der Fotodetektor vorzugsweise maschinenseitig angebracht sein, das gewandelte Signal wird über Lichtleiter zum Multipolstecker und von dort auf die Maschinenseite geleitet. Mit dieser Art des Wandlers können u. a. bestimmt werden: Verfärbung durch Temperatur, Druck, chemische Veränderung, pH, pO2, Glukosekonzentration (Hydrogel Optrode).

Einige oder alle erfindungsgemäßen Ausführungsformen können einen oder mehrere der oben oder im Folgenden genannten Vorteile aufweisen.

Moderne medizinische Systeme zur Blutbehandlung weisen üblicherweise eine Blutbehandlungsvorrichtung (auf der sogenannten "Maschinenseite") und daran anbringbare medizinische Einrichtungen (auf der sogenannten "Einrichtungsseite") auf. Ist die medizinische Einrichtung ein Einwegartikel oder Disposable so ist hierin auch von der sogenannten "Disposableseite" die Rede. Obgleich die vorliegende Erfindung nicht auf Disposables beschränkt ist, wird hierin exemplarisch für medizinische Einrichtungen auch auf Disposables Bezug genommen, ohne dass dies beschränkend zu verstehen ist. Die zu einer Blutbehandlungssitzung verwendeten Einwegartikel werden aus Gründen der Hygiene nach der Blutbehandlungssitzung entsorgt, da sie in Kontakt mit Blut des Patienten standen oder gestanden haben können.

Zur Überwachung der Behandlung sind Sensoren und Aktoren erforderlich, die auf dem Einwegartikel bzw. der medizinischen Einrichtung Parameter messen bzw. auf die Einrichtung einwirken.

Eine Funktionalisierung der Einwegartikel selbst, also die Integration der gesamten zum Überwachen eines konkreten Parameters jeweils erforderlichen Sensorik in den Einwegartikel, hat sich bisher zumeist als wirtschaftlich unrentabel herausgestellt, da die vergleichsweise teuren Sensorbauteile nach jeder Verwendung verworfen werden würden. Deshalb sind herkömmliche Einwegartikel zur medizinischen Blutbehandlung zumeist sehr einfach gestaltet, bestehen im Wesentlichen aus dem Schlauch zur Durchleitung des Bluts. Sensoren tragen sie aus dem vorstehend genannten Grund i. a. R. nicht.

Diese Sensoren und Aktoren sind im Stand der Technik folglich weitgehend maschinenseitig vorgesehen. Von dort wirken sie über eine Vielzahl von Sensor- und/oder Aktuatorschnittstellen auf die medizinische Einrichtung ein oder stehen mit dieser in Wechselwirkung. Diese Schnittstellen bestimmen in der Regel den Formfaktor und führen dazu, dass medizinische Systeme des Standes der Technik nicht beliebig klein ausgeführt werden können.

Die vorliegende Erfindung erlaubt es nun hingegen erstmals vorteilhaft, eine kostengünstige und damit wirtschaftliche Alternative zur Funktionalisierung von medizinischen Einrichtungen und auch Einwegartikeln bereit zu stellen. Sie erlaubt damit vorteilhaft die Miniaturisierung der eingesetzten Blutbehandlungssysteme. Deren Miniaturisierung ist nicht zuletzt deshalb wünschenswert, da dies zur Reduzierung sowohl der extrakorporalen Blutmenge als auch des Aufwandes für die extrakorporale Förderung und Hydraulik beiträgt.

Im Gegensatz zum Stand der Technik, in dem die Funktionalisierung durch die Integration der individuellen Sensorkomponenten in die Einwegartikel mit den vorstehend genannten Nachteilen versucht wurde, stellt die Erfindung, wie vorstehend beschrieben, eine Lösung zur wenig aufwändigen Verlagerung von Sensorkomponenten auf die medizinische Einrichtung dar.

Vorteilhaft ist ferner, dass mit der vorliegenden Erfindung eine Methode zur Verfügung gestellt wird, bei der alle einrichtungsseitig angeordneten Sensorkomponenten mit demselben Produktionsverfahren integriert werden können, was den Fertigungsaufwand weiter senken kann.

Die vorliegende Erfindung erlaubt dabei wahlweise eine vollständige Funktionalisierung oder eine nur teilweise Funktionalisierung der erfindungsgemäßen Einrichtung, die aufgrund der besonderen Art, wie die Wandler auf die Einrichtung verlagert werden, ohne nennenswerten wirtschaftlichen Verlust gemeinsam mit dem Wandler verworfen werden kann.

Unter einer teilweisen Funktionalisierung wird hierin beispielsweise im Falle eines Wandlers verstanden, dass die Kernaufgabe des Wandlers, also das Erfassen einer physikalischen, chemischen oder anderen Größe sowie das Wandeln dieser Größe in eine elektro-magnetisch übertragbare Substitutgröße, auf der medizinischen Einrichtung selbst, entweder invasiv (d. h. im Kontakt mit Blut oder einer Behandlungsflüssigkeit) oder nicht invasiv geschieht, jedoch z. B. die Verarbeitung des Signals sowie die Interpretation und weitere Schritte, die höherwertige z.B. integrierte elektronische Bauteile erfordert, auf Maschinenseite erfolgt. Ein Teil oder die gesamte Verarbeitung des Signals kann in einigen Fällen jedoch auch komplett auf der Einrichtungsseite erfolgen. Bei letzterer Ausführungsform der vorliegenden Erfindung wird von einer vollständigen Funktionalisierung gesprochen: wenigstens eine Sensoranordnung oder ein Sensor liegt vollständig additiv auf die Einrichtung aufgebracht auf letzterer vor. Dabei können die einrichtungsseitigen Komponenten einer Sensoranordnung oder eines Sensors erfindungsgemäß alle durch dasselbe maskenfreie und additive Produktionsverfahren aufgebracht werden, wie vorstehend ausgeführt.

Von Vorteil ist ferner, dass, wie erfindungsgemäß auch vorgeschlagen, Einwegartikel, wenn sie mit individuellen vollständigen Sensoren ausgestattet werden, somit vollständig funktionalisiert werden können. Das hierin vorgestellte Verfahren zum Aufbringen von Wandlern oder anderen Komponenten von Sensoren macht dies möglich.

Die teilweise Funktionalisierung und damit verbunden, der Verbleib der komplexen, zur Nachverarbeitung und Auswertung der Signale notwendigen Elektronik auf der Maschinenseite ist dadurch vorteilhaft, dass nur die zumeist einfacher gestalteten Komponenten der jeweiligen Sensoren auf der Einrichtung angeordnet sind, die durch die gemeinsame additive und maskierungsfreie Drucktechnik angebracht werden.

Die Funktionalisierung ist ferner wirtschaftlich von Vorteil, da die Herstellungskosten durch die gemeinsame Verwendung derselben Produktionstechnologie, idealerweise im selben Produktionsschritt für alle einrichtungsseitig angeordneten Sensorkomponenten, äußerst kostengünstig erfolgen kann.

Da das erfindungsgemäße Produktionsverfahren vorzugsweise ein additives, maskierungsfreies Druckverfahren ist, kann eine veränderte Version der Einrichtung, beispielsweise mit neuen Sensorgeometrien, optional sogar allein durch neues Aufspielen des entsprechenden Datensatzes in der Software produziert werden. Es ist keine Veränderung an der Produktionshardware notwendig, wie beispielsweise die Neuanschaffung eines Spritzgusswerkzeugs, das in der Produktion der medizinischen Einrichtung mittels Spritzguss erforderlich wäre. Auch dieser Aspekt führt zu einer vereinfachten Fertigung und zu verbesserter Wirtschaftlichkeit.

Ein weiterer Vorteil der Erfindung zu herkömmlichen (nicht-funktionalisierten) Systemen besteht darin, dass die zu messende physikalische oder chemische Größe nicht mechanisch auf die Maschinenseite geführt werden muss, wie das beispielsweise bei bekannten Drucksensoren der Fall ist, bei denen die zu messenden Druckverhältnisse über eine pneumatische Leitung zu einer Messmembran geführt werden, welche nicht *disposable* ist und maschinenseitig angeordnet ist. Um diese vor Flüssigkeit zu schützen, sind regelmäßig aufwändige Schutzmembranen notwendig, welche als Transducerprotector (TP) bezeichnet werden. Erfindungsgemäß können also alle mechanischen Schnittstellen zur Maschine entfallen. Alle zu messenden Größen können mittels der integrierten Teile der Sensorik zumindest in analoge elektromagnetische Signale (Strom, Spannung, optische Signale) gewandelt und erst in dieser Form zur Verarbeitungs- und Auswerteeinheit auf der Maschinenseite übermittelt werden. Zur Ankopplung der gesamten einrichtungsseitigen Sensorik ist nur noch eine entsprechende mehrkanalige elektromagnetische und/oder optische Schnittstelle notwendig (Multipolstecker), was eine signifikante Verkleinerung derselben erlaubt.

Die vorliegende Erfindung erlaubt ferner eine optimale Übermittlung des Signals vom Wandler des Sensors an z. B. einen Multipolstecker durch Unterscheidung verschiedener Anschluss- bzw. Leitungssegmente und der Verwendung der bezüglich Leitungssicherheit, Produktionsgeschwindigkeit und Kosten optimalen Technik zum Aufbringen der Leiterbahnen. Weiterhin stellt die Erfindung die optimale Konnektierung zwischen den einzelnen Leitungssegmenten zur Verfügung.

Im Folgenden wird die vorliegende Erfindung unter Bezugnahme auf die beigefügte Zeichnung beschrieben. In der Zeichnung bezeichnen identische Bezugszeichen gleiche oder identische Elemente. Es zeigen:
- Fig. 1: ein medizinisches System zur Blutbehandlung mit einer medizinischen Einrichtung gemäß der vorliegenden Erfindung als teilfunktionalisiertes Disposable;
- Fig. 2: eine medizinische Einrichtung gemäß einer zweiten beispielhaften Ausführungsform der vorliegenden Erfindung;
- Fig. 3: zwei Wandler, welche mittels eines maskierungsfreien und additiven Verfahrens dreidimensional entlang der Innenkontur eines Hartteils einer erfindungsgemäßen Einrichtung aufgebracht sind;
- Fig. 4: einen Ausschnitt eines Hartteils der erfindungsgemäßen Einrichtung in einer weiteren exemplarischen Ausführungsform mit zwei Vergrößerungen hiervon;
- Fig. 5a: zeigt beispielhaft eine Maschinenschnittstelle zur Verbindung mit der erfindungsgemäßen Einrichtung, hier exemplarisch als zangenartiger oder klemmenartiger Verbinder ausgeführt;
- Fig. 5b: ein als planarer Multipolstecker ausgeführtes Gegenstück der Maschinenschnittstelle der Fig. 5a;
- Fig. 5c: ein als 3-dimensionaler Multipolstecker ausgeführtes Gegenstück der Maschinenschnittstelle der Fig. 5a;
- Fig. 6a: eine Kreuzung oder Überlagerung von zwei Leitungen der erfindungsgemäßen Einrichtung; und,
- Fig. 6b: einen vergrößerten Ausschnitt der Darstellung der Fig. 6a im Schnitt.

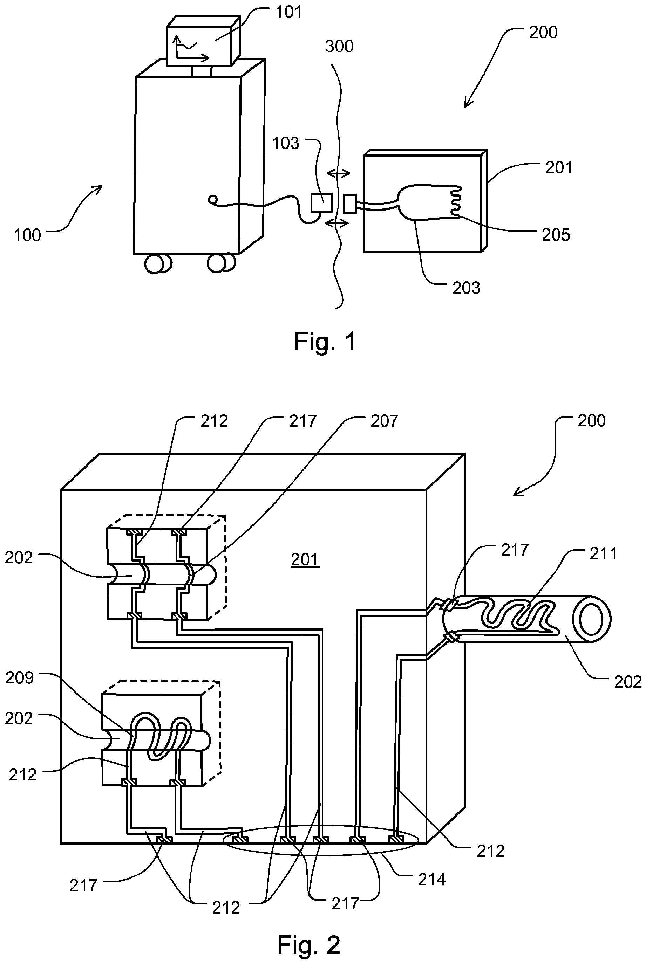

**Fig. 1** zeigt sehr schematisch und nur beispielhaft eine Blutbehandlungsvorrichtung 100 mit einer erfindungsgemäßen medizinischen Einrichtung 200 (kurz: Einrichtung 200).

Die Einrichtung 200 ist exemplarisch als ein Disposable ausgestaltet. Sie wurde mittels eines additiven, maskierungsfreien Druckverfahrens teilfunktionalisiert.

Die Blutbehandlungsvorrichtung 100 und die erfindungsgemäße Einrichtung 200 sind über eine Schnittstelle 300 miteinander in Signalkommunikation verbunden.

Die Einrichtung 200 weist ein Hartteil 201 auf. Auf dem Hartteil 201 sind Teile einer Sensoranordnung vorgesehen, hier eine Leiterbahn 203 und ein Wandler 205.

Der Wandler 205 kann in seiner Gesamtheit als erster Abschnitt im Sinne der vorliegenden Erfindung verstanden werden, der mit einem ersten Aufbringungsverfahren aufgebracht ist.

Die Leiterbahn 203 kann in ihrer Gesamtheit als zweiter Abschnitt im Sinne der vorliegenden Erfindung verstanden werden, die mit einem zweiten Aufbringungsverfahren aufgebracht ist.

Der Wandler 205 kann beispielsweise ein Druckaufnehmer sein. Er kann auf das Hartteil 201 aufgedruckt sein. Er kann disposableseitig lediglich die zu messende Größe Druck in ein analoges elektrisches Signal wandeln.

Das elektrische Signal wird über die ebenfalls additiv aufgedruckte Leiterbahn 203 zu der definierten Schnittstelle 300 geleitet, welche in Verbindung mit einer maschinenseitigen Auswerteeinheit, angedeutet durch einen Monitor 101 zur Darstellung der mittels der Auswerteeinheit erzielten Auswerteergebnisse, steht.

Maschinenseitig kann das Signal mittels eines AD (Analog-Digital-)-Wandlers oder AD-Converters (kurz: ADC) 103 digitalisiert werden, es können Schritte der Nachverarbeitung (Filter, Glättung, Fouriertransformation, Zero-Filling etc.) erfolgen, bevor schließlich eine Auswertung und Interpretation erfolgt. Alle diese optionalen Schritte können z. B. in der Auswerteeinheit durchgeführt werden.

**Fig. 2** zeigt eine medizinische Einrichtung 200 gemäß einer zweiten beispielhaften Ausführungsform der vorliegenden Erfindung.

Die medizinische Einrichtung 200 ist hier wiederum ein teilfunktionalisiertes Disposable, mit drei unterschiedlichen Wandlern 207, 209 und 211.

Dabei zeigt Fig. 2 wie auch die folgenden Figuren je ein Beispiel von aufgebrachten Wandlern 207, 209 und 211 einerseits und Leiterbahnen 212 andererseits, wobei Wandler und Leiterbahn mittels verschiedener Aufbringungsverfahren aufgebracht wurden oder erfindungsgemäß aufgebracht worden sein können - alternativ können sowohl wenigstens einer der Wandler 207, 209 und 211 einerseits und eine der Leiterbahnen 212 andererseits jeweils mittels mehr als eines Aufbringungsverfahrens - als auch wenigstens mittels eines ersten und eines zweiten Aufbringungsverfahrens - aufgebracht worden sein.

Oben links ist in Fig. 2 beispielhaft ein Wandler 207 zur Messung der Leitfähigkeit angeordnet. In der vorliegenden, vereinfachten Darstellung besteht dieser aus zwei Leiterbahnen, welche erfindungsgemäß mittels des additiven, maskierungsfreien Druckverfahrens im Inneren eines fluidführenden Kanals 202 der Einrichtung 200 aufgebracht sind.

Die beiden anderen Wandler 209, 211 zeigen beispielhafte Ausführungen von Druckaufnehmern. Diese können als Dehnungsmessstreifen an der Innenkontur (siehe den Wandler 209) oder an der Außenkontur (siehe den Wandler 211) des fluidführenden Kanals oder Schlauchs 202 der Einrichtung 200 angebracht sein.

Über die Wandler 209, 211 hinaus zeigt Fig. 2 beispielhaft die elektromagnetische Signalführung mit Leiterbahnen 212 und Kontakte 217 zur Maschinenschnittstelle 300, hier beispielhaft einen Multipolstecker 214. Die Leiterbahnen 212 zur Signalleitung können hierbei mit demselben additiven und maskierungsfreien Verfahren, insbesondere im selben Verfahrensschritt, auf die planare Fläche des Hartteils 201 aufgebracht sein.

Es sind aber auch nicht-planare (dreidimensionale) Leitungsführungen im oben beschriebenen Sinn oder Kreuzungen von (entsprechend isolierten) Leitungen möglich. Die Leiterbahnen 212 sowie die Kontakte 217 können auch mittels einer zweiten, ebenfalls additiven und maskierungsfreien Drucktechnik aufgebracht werden, z. B. in einem zweiten Produktionsschritt, der dem Aufbringen der Wandler 207, 209, 211 folgt. Die Gesamtheit der Leiterbahnen 212 zur Signalführung vom Ort des jeweiligen Wandlers 207, 209, 211, welcher die zu messende Größe z. B. in ein elektromagnetisches Signal wandelt, das mittels der Leiterbahnen 212 zur Maschinenschnittstelle 300 geführt wird, kann auch einzelne Teile aus oder mit leitfähigen Polymeren beinhalten, die mittels Zweikomponenten-Spritzguss aufgebracht sind oder wurden.

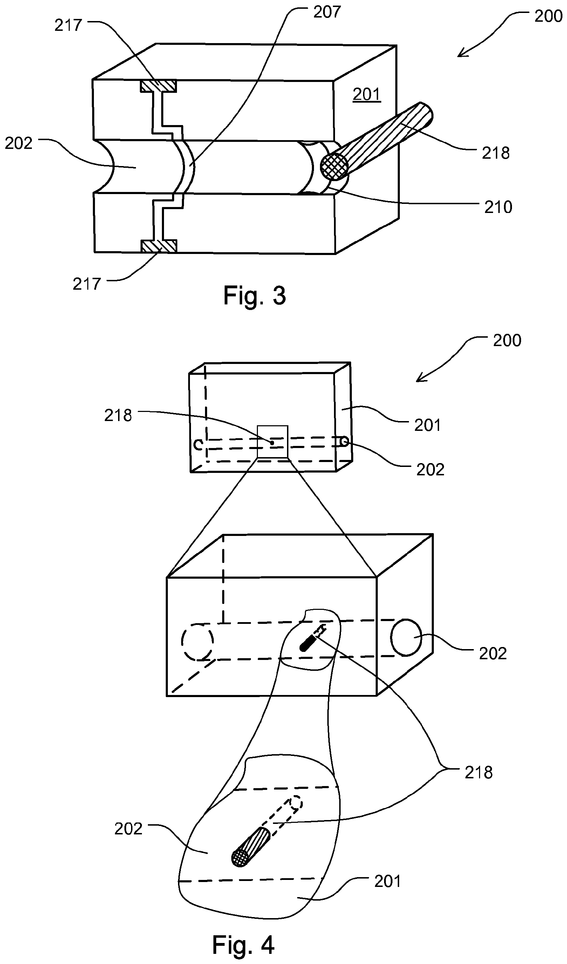

**Fig. 3** zeigt zwei Wandler 207, 210, welche jeweils mittels eines oder mehrerer maskierungsfreien und additiven Verfahren dreidimensional entlang der Innenkontur einer Fluidbahn 202 in Form eines Kanals im Hartteil 201 der medizinischen Einrichtung 200 aufgebracht wurden.

Die beiden Wandler 207, 210 können beispielsweise Elektroden eines gemeinsamen Leitfähigkeitssensors sein. Sie könnten auch Wandler unterschiedlich messender Sensoren sein.

Fig. 3 zeigt nun zwei verschiedene Möglichkeiten, die jeweiligen Wandler 207, 210 bzw. deren Elektroden leitend anzukoppeln, d. h. sie elektrisch mit einer weiterführenden Leiterbahn zu verbinden.

Eine solche weiterführende Leiterbahn ist typischerweise die flächige Leiterbahn des zweiten Leitungssegments - hier nicht dargestellt. Die Elektrode auf der linken Seite ist mit demselben Verfahren angekoppelt, mit dem sie selbst auch auf die Innenkontur des Kanals aufgebracht wurde. Dies kann beispielsweise durchgeführt werden, indem beim offenen Kanal einer Kassettenhälfte - wie dargestellt - die Enden der Elektrode über die Kanalkante bis auf die Kassettenoberfläche geführt werden.

Im Beispiel auf der rechten Seite der Fig. 3 ist die Ankopplung des Wandlers 210 mittels einer sogenannten Durchkontaktierung gezeigt. Hierzu wird ein Kontaktstift 218 - in Fig. 4 vergrößert dargestellt - durch einen Abschnitt des Hartteils 201, z. B. durch eine Blutkassettenhälfte, hindurch geführt. Auf der einen Seite des Hartteils 201 ist der Kontaktstift 218 mit dem Wandler 210 leitend verbunden. Auf der anderen Seite des Hartteils 201 führt der Kontaktstift 218 zur flächigen Verbindung des zweiten Leitungssegments (hier nicht dargestellt).

Der Kontaktstift 218 kann in einer Ausführungsform ein Metallstift oder ein anderer Metallkörper sein, der bei der Herstellung des Hartteils 201 mittels Spritzgussverfahren umspritzt bzw. eingespritzt wird.

Der Kontaktstift 218 kann alternativ selbst mittels Spritzguss hergestellt sein, z. B. in einem zweiten Spritzschritt. Bei diesem sogenannten Zweikomponentenspritzguss wird beim Spritzen des Kontaktstifts 218 ein leitfähiges Material verwendet, etwa leitfähige Polymere oder mit metallischen Partikeln angereicherte Compoundmaterialien. Der Kontaktstift 218 kann schließlich auch nachträglich in einen dafür vorgesehenen Durchgang eingelegt werden.

**Fig. 4** zeigt die hierin als Durchkontaktierung bezeichnete Ausführungsform in mehrfacher Vergrößerung. Sie zeigt die Durchkontaktierung exemplarisch mittels Zweikomponentenspritzguss ermöglicht.

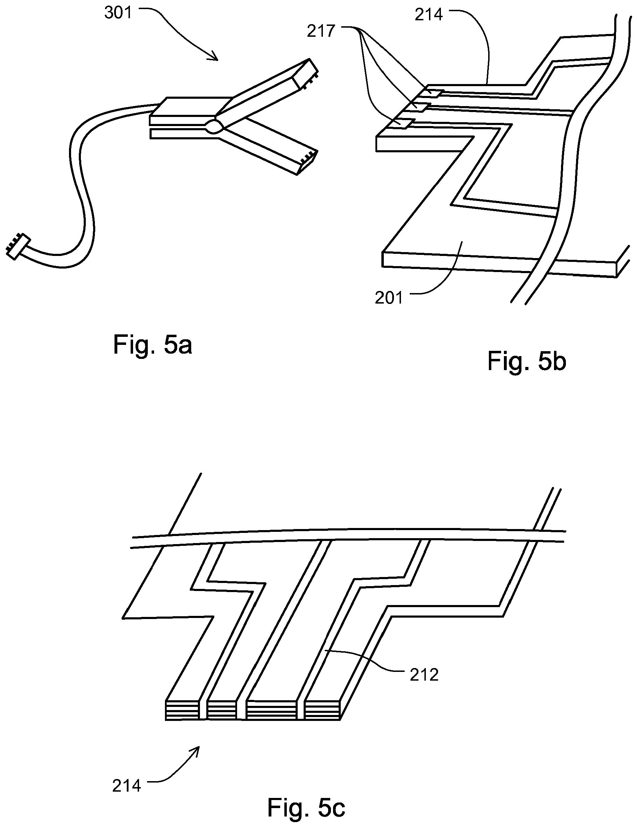

**Fig. 5a** zeigt beispielhaft die Maschinenschnittstelle 300, hier exemplarisch als zangenartiger oder klemmenartiger Verbinder in Gestalt einer Zange 301 ausgeführt. Der Verbinder, der auch als Steckverbinder ausgestaltet sein könnte, ist dazu eingerichtet, bei seinem Anlegen an die entsprechenden Kontakte auf der Einrichtung 200 (siehe z. B. Fig. 3) eine leitende Verbindung zur nicht gezeigten Kontrolleinheit oder Auswerteeinheit der Blutbehandlungsvorrichtung 100 herzustellen. Über die leitende Verbindung können z. B. analoge, elektro-magnetisch übermittelbare Signale von der erfindungsgemäß teilfunktionalisierten Einrichtung 200 zur Blutbehandlungsvorrichtung 100 geleitet werden.

**Fig. 5b** zeigt ein mögliches Gegenstück zur Zange 301 der Fig. 5a auf der Seite der Einrichtung 200 in unterschiedlichen Ansichten. In Fig. 5b ist beispielhaft ein flächiger Multipolstecker 214 gezeigt, d. h. die Leiterbahnen 212 sind ausschließlich auf der Oberfläche des Multipolsteckers 214 oder des Hartteils 201 im Bereich des Mulitpolsteckers 214 aufgedruckt. Der Multipolstecker 214 ist somit planar aufgebracht, d. h. 2-dimensional. Er kann sowohl mittels eines maskierungsfreien, additiven Verfahrens aufgebracht sein, aber auch mittels klassischer Verfahren wie Siebdruck o. ä.

**Fig. 5c** zeigt den Multipolstecker 214 in einer Ausführungsform mit dreidimensionaler Bedruckung, d.h. hier werden die Leiterbahnen um die Kante an der Stirnseite herumgeführt und bedecken auch die (hier exemplarisch gesamte) Stirnseite des den Multipolstecker 214 tragenden Abschnitts des Hartteils 201.

Diese Ausführungsform kann insbesondere für eine verbesserte Kontaktsicherheit sorgen, bei mechanisch beanspruchten Situationen, wo eine versehentliche Diskonnektion droht. Die dreidimensionale Bedruckung dieser Ausführungsform erfolgt vorzugsweise mit einem maskierungsfreien, additiven Verfahren.

Die Ankopplung des Multipolsteckers 214 an das zweite Leitungssegment, also die flächige Verbindung, die das Signal vom Wandler kommend zuvor durchläuft, kann wie zuvor durch Aufdrucken einer direkten Verbindung ausgeführt sein. Weiterhin ist in bestimmten Situationen auch die zuvor erläuterte Ankopplung mittels Durchkontaktierung möglich.

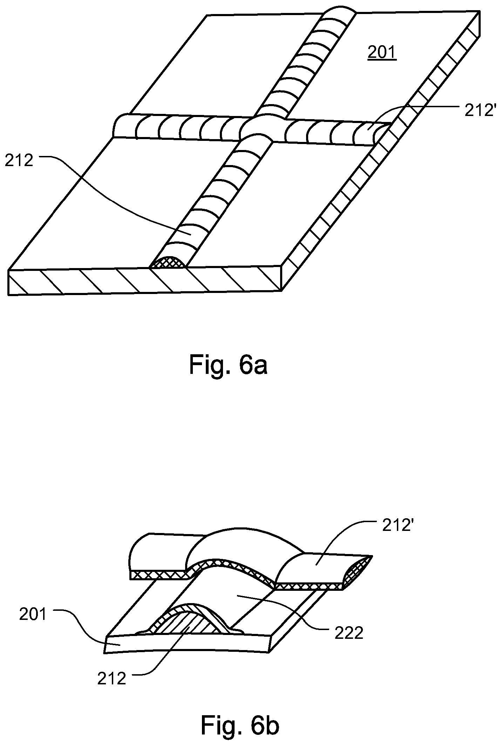

**Fig. 6a** und **Fig. 6b** zeigen einen weiteren Aspekt der Erfindung, der vorzugsweise in z. B. dem o. g. zweiten Leitungssegment und/oder bei flächigen Leiterbahnen Anwendung finden wird.

In diesen hier als flächig bezeichneten Verbindungen werden die Signale der einzelnen, in Fig. 6 nicht gezeigten Wandler der Einrichtung 200 über oder unter der Oberfläche der Einrichtung 200 zum Multipolstecker 214 geführt, von wo sie zur Kontroll- oder Auswerteeinheit der Blutbehandlungsvorrichtung übermittelt werden können. Je nach Topologie der Oberfläche und der Belegung der einzelnen Kontakte des Multipolsteckers 214 kann es erforderlich sein, dass sich einzelne Leitungen 212, 212' oder Verbindungen kreuzen.

Eine solche Kreuzung oder Überlagerung 220 ist in Fig. 6a und erneut in einem vergrößerten Ausschnitt im Schnitt in Fig. 6b gezeigt.

Eine solche Kreuzung oder Überlagerung 220 kann vorzugsweise in z. B. dem o. g. zweiten Leitungssegment und/oder bei flächigen Leiterbahnen vorgesehen sein. Dort kann sie mit vergleichsweise geringem Aufwand umgesetzt werden, da in der flächigen Verbindung am meisten Platz ist.

Sich kreuzende Leiterbahnen 212, 212' (kurz: Leiter) sind mit dem erfindungsgemäßen Verfahren vorteilhaft einfach zu realisieren, da sie z. B. mittels des hierin genannten maskierungsfreien, additiven Bedruckens schichtweise erstellt werden können.

Prinzipiell können beide Leiterbahnen 212, 212' nacheinander auf die Oberfläche der Substratschicht aufgebracht werden. Damit die elektromagnetischen Signale nicht durch Kurzschluss gestört werden, kann zwischen den beiden Leitern eine Isolierschicht 222 aufgetragen werden.

Hierzu kann die gesamte erste Leiterbahn 212 mit einer Isolierschicht 222 über- oder nachgedruckt werden, so dass sie in ihrer gesamten Länge zur Oberfläche elektrisch isoliert ist.

Alternativ kann die Isolierung auch ausschließlich am unmittelbaren Ort der Kreuzung der beiden Leitungen 212, 212' erfolgen.

Wenn die Einrichtung 200 nicht in einem späteren Produktionsschritt durch Verbinden mit einer zweiten Einrichtungshälfte, etwa einer zweiten Kassettenhälfte, geschlossen wird, d.h. die zweite Leitung 212' der sich kreuzenden Leitungen frei liegt, kann auch diese in einem zweiten oder weiteren Bedruckungsschritt mit einer solchen Isolierschicht bedruckt werden, um das übermittelte Signal gegen Kurzschlüsse zu schützen.

Um das Signal ferner gegen induktive oder kapazitive Kopplungen durch elektromagnetische Störsignale zu schützen, kann auch um die äußere Isolierschicht erneut eine leitende Schicht (nicht in den Figuren gezeigt) gedruckt werden, welche auf ein Abschirmungspotential gelegt wird.

Alternativ kann auch jede der beiden sich kreuzenden Leitungen 212, 212' außerhalb ihrer Isolierschicht 222 eine solche Abschirmungsschicht aufweisen. Jede der Abschirmungsschichten kann nach außen erneut durch eine weitere Isolierschicht elektrisch isoliert werden. Dies ist insbesondere bei der inneren Masseschicht vorteilhaft, wenn darüber die zweite, kreuzende Leitung 212' zum Signaltransport angeordnet ist.

### Bezugszeichenliste

- 100: Blutbehandlungsvorrichtung
- 101: Monitor
- 103: AD-Wandler

- 200: Blutkassette als Beispiel einer medizinischen Einrichtung
- 201: Kassettenkörper oder Kassettengrundkörper, Hartteil; Hartkörper, Substrat
- 202: Fluidbahn, Kanal, Strömungskanal
- 203: Leiterbahn, Leiter oder Signalleiter
- 205: Wandler
- 207: Wandler
- 209: Wandler
- 210: Wandler
- 211: DMS-Element als Wandler
- 212: Leiterbahn, Leiter oder Signalleiter
- 212': Leiterbahn, Leiter oder Signalleiter
- 214: Multipolstecker, Multipolverbinder
- 217: Kontakt
- 218: Kontaktstift
- 220: Kreuzung oder Überlagerung von Leiterbahnen
- 222: Isolator; Isolierschicht

- 300: Schnittstelle, Maschinenschnittstelle
- 301: Zange

## Patentansprüche

1. Medizinische Einrichtung (200) mit jeweils wenigstens
- einem Hartteil (201) mit Fluidbahnen (202) zum Führen eines medizinischen Fluids, insbesondere Blut, durch das Hartteil (201);
- einem Wandler (205, 207, 209, 210, 211), wobei der Wandler (205, 207, 209, 210, 211) angeordnet ist zum Messen einer Eigenschaft des medizinischen Fluids, während dieses in einer der Fluidbahnen (202) vorliegt; und
- einer Leiterbahn (212);
**dadurch gekennzeichnet, dass** wenigstens ein erster Abschnitt des Wandlers (205, 207, 209, 210, 211) oder der Leiterbahn (212) mittels eines ersten additiven Aufbringungsverfahrens auf das Hartteil (201) aufgebracht ist;
wobei wenigstens ein zweiter Abschnitt des Wandlers (205, 207, 209, 210, 211) oder der Leiterbahn (212) mittels eines zweiten additiven Aufbringungsverfahrens auf das Hartteil (201) aufgebracht ist; und
wobei sich das erste additive und das zweite additive Aufbringungsverfahren voneinander unterscheiden.

2. Einrichtung nach Anspruch 1, wobei das erste, das zweite oder beide Aufbringungsverfahren das Aufbringen von leitfähiger Tinte umfassen.

3. Einrichtung nach Anspruch 1 oder 2, wobei das erste, das zweite oder beide Aufbringungsverfahren ein maskierungsfreies Aufbringen umfassen.

4. Einrichtung nach einem der vorangegangenen Ansprüche, wobei die medizinische Einrichtung (200) eine Vielzahl von Wandlern (205, 207, 209, 210, 211) aufweist, welche jeweils zumindest in einem Abschnitt hiervon mittels additiven Aufbringungsverfahrens, vorzugsweise eines Druckverfahrens, auf das Hartteil (201) aufgebracht sind und/oder wobei die Einrichtung wenigstens einen Multipolstecker (214) aufweist, welcher mittels des ersten, zweiten oder eines dritten additiven Aufbringungsverfahren aufgebracht wurde.

5. Einrichtung nach einem der vorangegangenen Ansprüche, wobei der oder die Wandler (205, 207, 209, 210, 211) beispielsweise zum Messen oder Bestimmen von Leitfähigkeit, Druck, Spannung oder Strom konfiguriert oder ausgestaltet sind.

6. Einrichtung nach einem der vorangegangenen Ansprüche, wobei wenigstens ein elektrisch leitender Kontaktstift (218) im Hartteil (201) vorliegt, wobei der Kontaktstift (218) in elektrischer Leitverbindung mit dem Wandler (205, 207, 209, 210, 211) oder der Leiterbahn (212) steht.

7. Einrichtung nach einem der vorangegangenen Ansprüche, wobei die medizinische Einrichtung (200) eine Blutkassette ist.

8. Verfahren zum Herstellen einer medizinischen Einrichtung (200) nach einem der vorangegangenen Ansprüche, mit den Schritten:
- Herstellen oder Bereitstellen eines Hartteils (201) der medizinischen Einrichtung (200) mit einem Fluidsystem für ein medizinisches Fluid;
- Aufbringen wenigstens eines ersten Abschnitts eines Wandlers (205, 207, 209, 210, 211) oder einer Leiterbahn (212, 212') mittels eines ersten additiven Aufbringungsverfahrens, vorzugsweise eines Druckverfahrens, auf das Hartteil (201);
- Aufbringen wenigstens eines zweiten Abschnitts des Wandlers (205, 207, 209, 210, 211) oder der Leiterbahn (212, 212') mittels eines zweiten additiven Aufbringungsverfahrens auf das Hartteil (201); und
wobei sich das erste additive und das zweite additive Aufbringungsverfahren voneinander unterscheiden.

9. Verfahren nach Anspruch 8, wobei das erste, das zweite oder beide Aufbringungsverfahren das Aufbringen von leitfähiger Tinte umfassen.

10. Verfahren nach Anspruch 8 oder 9, wobei das erste, das zweite oder beide Aufbringungsverfahren ein maskierungsfreies Aufbringen sind oder umfassen.

11. Verfahren nach einem der Ansprüche 8 bis 10, wobei wenigstens ein Multipolstecker (214) mittels des ersten, zweiten oder eines dritten additiven Aufbringungsverfahrens aufgebracht wird.

12. Verfahren nach einem der Ansprüche 8 bis 11, umfassend wenigstens einen der folgenden Schritte:
- Nachbearbeitungsschritte, insbesondere an Leiterbahnen (212, 212'), wie z. B. Schleifen, Polieren, Isolieren, Aufbringen weiterer funktionaler Schichten aus anderem Material;
- additives Aufbringen, z. B. durch Aufdrucken, der Signalanbindung an die Maschinenschnittstelle (300);
- additives Aufbringen, z. B. durch Aufdrucken, des Multipolsteckers (214);
- Zusammenfügen von zwei oder mehr mittels additiven Aufbringens bearbeiteter Abschnitte der medizinischen Einrichtung (200).

13. Verfahren nach einem der Ansprüche 8 bis 12, umfassend wenigstens einen der folgenden Schritte:
- Einbringen eines Kontaktstifts (218) während oder nach einem Spritzgussverfahren, mit welchem das Hartteil (201) gefertigt wurde, in das Hartteil (201).

14. Verfahren nach einem der Ansprüche 8 bis 13, umfassend die folgenden Schritte:
- Aufbringen von wenigstens zwei Leiterbahnen (212, 212'), die sich in jeweils wenigstens einem Abschnitt kreuzen; und
- Aufbringen einer Isolierschicht (222) zwischen den beiden Leiterbahnen (212, 212').

15. Verfahren nach einem der Ansprüche 8 bis 14, umfassend den folgenden Schritt:
- Aufbringen einer Abschirmschicht auf wenigstens einer der Leiterbahnen (212, 212').

## Claims

1. A medical device (200) comprising at least, respectively:
- a hard part (201) with fluid paths (202) for guiding a medical fluid, in particular blood, through the hard part (201);
- a transducer (205, 207, 209, 210, 211),
the transducer (205, 207, 209, 210, 211) being arranged to measure a property of the medical fluid when it is present in one of the fluid paths (202), and
- a conductor path (212);
wherein at least a first section of the transducer (205, 207, 209, 210, 211) or of the conductor path (212) is applied to the hard part (201) by means of a first additive application method;
**characterized in that** at least a second section of the transducer (205, 207, 209, 210, 211) or of the conductor path (212) is applied to the hard part (201) by means of a second additive application method; and
wherein the first additive application method and the second additive application method differ from each other.

2. The device according to claim 1, wherein the first, the second or both application methods includes/e the application of conductive ink.

3. The device according to claims 1 or 2, wherein the first, the second or both application methods includes/e a mask-free application.

4. The device according to anyone of the preceding claims wherein the medical device (200) comprises a plurality of transducers (205, 207, 209, 210, 211), each of which is respectively, at least in one section thereof, applied to the hard part (201) by means of an additive application method, preferably by means of a printing method, and/or wherein the device comprises at least one multi-pole plug (214), which has been applied by means of the first, the second or a third additive application method.

5. The device according to anyone of the preceding claims wherein the transducer or the transducers (205, 207, 209, 210, 211) is/are configured or designed for example to measure or determine conductivity, pressure, voltage or current.

6. The device according to anyone of the preceding claims wherein at least one electrically conductive contact pin (218) is present in the hard part (201), the contact pin (218) being in electrical conductive connection with the transducer (205, 207, 209, 210, 211) or with the conductor path (212).

7. The device according to anyone of the preceding claims wherein the medical device (200) is a blood cassette.

8. A method for producing a medical device (200) according to anyone of the preceding claims, comprising the following steps:
- producing or providing a hard part (201) of the medical device (200) with a fluid system for a medical fluid;
- applying at least a first section of a transducer (205, 207, 209, 210, 211) or of a conductor path (212, 212') to the hard part (201) by means of a first additive application method, preferably a printing method;
- applying at least a second section of the transducer (205, 207, 209, 210, 211) or of the conductor path (212, 212') to the hard part (201) by means of a second additive application method; and
wherein the first additive application method and the second additive application method differ from each other.

9. The method according to claim 8, wherein the first, the second or both application methods includes/e the application of conductive ink.

10. The method according to claim 8 or 9, wherein the first, the second or both application methods is/are or includes/e a mask-free application.

11. The method according to anyone of the claims 8 to 10, wherein at least a multi-pole plug (214) is applied by means of the first, the second or a third additive application method.

12. The method according to anyone of the claims 8 to 11 comprising at least one of the following steps:
- post-processing steps, in particular on conductor paths (212, 212'), such as for example grinding, polishing, isolating, applying further functional layers made of other material;
- additive application, for example by printing, of the signal connection to the machine interface (300);
- additive application, for example by printing, of the multi-pole plug (214);
- assembly of two or more sections of the medical device (200) processed by means of additive application.

13. The method according to anyone of claims 8 to 12, comprising at least one of the following steps:
- introducing a contact pin (218) into the hard part (201) during or after an injection molding process with which the hard part (201) was manufactured.

14. The method according to anyone of claims 8 to 13, comprising the following steps:
- applying at least two conductor paths (212, 212'), crossing each other in at least one section; and
- applying an insulating layer (222) between the two conductor paths (212, 212').

15. The method according to anyone of claims 8 to 14, comprising the following step:
- applying a shielding layer on at least one of the conductor paths (212, 212').

## Revendications

1. Un dispositif médical (200) comprenant respectivement au moins:
- une partie rigide (201) avec des passages pour fluide (202) prévus pour guider un fluide médical, notamment du sang, au travers de la partie rigide (201);
- un transducteur (205, 207, 209, 210, 211),
le transducteur (205, 207, 209, 210, 211) étant agencé de façon à mesurer une propriété du fluide médical quand celui-ci est présent dans l'un des passages pour fluide (202), et
- une piste conductrice (212);
où au moins une première section du transducteur (205, 207, 209, 210, 211) ou de la piste conductrice (212) est appliquée sur la partie rigide (201) au moyen d'un premier procédé d'application additive;
**caractérisé en ce qu'**au moins une seconde partie du transducteur (205, 207, 209, 210, 211) ou de la piste conductrice (212) est appliquée sur la partie rigide (201) au moyen d'un second procédé d'application additive; et
où le premier procédé d'application additive et le second procédé d'application additive diffèrent l'un de l'autre.

2. Le dispositif selon la première revendication, où le premier, le second, ou les deux procédés d'application comprend/nent l'application d'une encre conductrice.

3. Le dispositif selon la revendication 1 ou 2, où le premier, le second, ou les deux procédés d'application comprend/nent une application sans masquage.

4. Le dispositif selon l'une quelconque des revendications précédentes, où le dispositif médical (200) comprend une pluralité de transducteurs (205, 207, 209, 210, 211), où chacun, au moins dans une section de celui-ci, est appliqué sur la partie rigide (201) au moyen d'un procédé d'application additive, de préférence au moyen d'un procédé d'impression, et/ou où le dispositif comprend au moins une fiche multi-pôles (214) qui a été appliquée au moyen du premier, du second ou d'un troisième procédé d'application additive.

5. Le dispositif selon l'une quelconque des revendications précédentes où le ou les transducteur/s (205, 207, 209, 210, 211) est/sont configuré/s ou conçu/s par exemple pour mesurer ou déterminer la conductivité, la pression, la tension ou le courant.

6. Le dispositif selon l'une quelconque des revendications précédentes, où au moins une broche de contact électriquement conductrice (218) est présente dans la partie rigide (201), la broche de contact (218) étant en connexion électriquement conductrice avec le transducteur (205, 207, 209, 210, 211) ou la piste conductrice (212).

7. Le dispositif selon l'une quelconque des revendications précédentes, où le dispositif médical (200) est une cassette à sang.

8. Un procédé pour fabriquer un dispositif médical (200) selon l'une des revendications précédentes, comprenant les étapes suivantes:
- la fabrication ou la fourniture d'une partie rigide (201) du dispositif médical (200) avec un système fluidique pour un fluide médical;
- l'application d'au moins une première section d'un transducteur (205, 207, 209, 210, 211) ou d'une piste conductrice (212, 212') sur la partie rigide (201) au moyen d'un premier procédé d'application additive, de préférence d'un procédé d'impression;
- l'application d'au moins une seconde section du transducteur (205, 207, 209, 210, 211) ou de la piste conductrice (212, 212') sur la partie rigide (201) au moyen d'un second procédé d'application additive; et
où le premier procédé d'application additive et le second procédé d'application additive diffèrent l'un de l'autre.

9. Le procédé selon la revendication 8, où le premier, le second, ou les deux procédés d'application comprend/nent l'application d'une encre conductrice.

10. Le procédé selon la revendication 8 ou 9, le premier, le second, ou les deux procédés d'application est/sont ou comprend/nent une application sans masquage.

11. Le procédé selon l'une quelconque des revendications 8 à 10, où au moins une fiche multi-pôles (214) est appliquée au moyen du premier, du second, ou d'un troisième procédé d'application additive.

12. Le procédé selon l'une quelconque des revendications 8 à 11 comprenant au moins une des étapes suivantes:
- des étapes de post-traitement, en particulier sur les pistes conductrices (212, 212'), comme par exemple le meulage, le polissage, l'isolation, l'application de couches fonctionnelles supplémentaires d'un autre matériau;
- l'application additive, par exemple par impression, de la connexion du signal à l'interface de la machine (300);
- l'application additive, par exemple par impression, de la fiche multi-pôles (214);
- l'assemblage de deux ou de plusieurs sections du dispositif médical (200) traitées au moyen d'une application additive.

13. Le procédé selon l'une quelconque des revendications 8 à 12, comprenant au moins une des étapes suivantes:
- l'insertion d'une broche de contact (218) dans la partie rigide (201) pendant ou après un processus de moulage par injection avec lequel la partie rigide (201) a été fabriquée.

14. Le procédé selon l'une quelconque des revendications 8 à 13, comprenant les étapes suivantes:
- l'application d'au moins deux pistes conductrices (212, 212'), chacune se croisant dans au moins une section; et
- l'application d'une couche isolante (222) entre les deux pistes conductrices (212, 212').

15. Le procédé selon l'une quelconque des revendications 8 à 14, comprenant l'étape suivante:
- l'application d'une couche de blindage sur au moins une des pistes conductrices (212, 212').
